## Europäisches Patentamt
## European Patent Office
## Office européen des brevets

(19)

(11) Veröffentlichungsnummer: **0 038 484**
**B1**

(12)

# EUROPÄISCHE PATENTSCHRIFT

(45) Veröffentlichungstag der Patentschrift:
**14.12.83**

(51) Int. Cl.³: **A 61 N 1/04**

(21) Anmeldenummer: **81102709.3**

(22) Anmeldetag: **09.04.81**

(54) **Endocard-Elektrodenanordnung.**

(30) Priorität: **21.04.80 DE 3015260**

(43) Veröffentlichungstag der Anmeldung:
**28.10.81 Patentblatt 81/43**

(45) Bekanntmachung des Hinweises auf die Patenterteilung:
**14.12.83 Patentblatt 83/50**

(84) Benannte Vertragsstaaten:
**DE FR NL SE**

(56) Entgegenhaltungen:
**FR - A - 2 122 032**
**FR - A - 2 133 843**
**FR - A - 2 257 262**
**FR - A - 2 345 167**
**FR - A - 2 457 697**
**GB - A - 1 079 768**
**US - A - 4 156 429**

(73) Patentinhaber: **Siemens-Elema AB, Röntgenvägen 2,
S-171 95 Solna 1 (SE)**

(84) Benannte Vertragsstaaten: **SE**

(73) Patentinhaber: **SIEMENS AKTIENGESELLSCHAFT,
Berlin und München Wittelsbacherplatz 2,
D-8000 München 2 (DE)**

(84) Benannte Vertragsstaaten: **DE FR NL**

(72) Erfinder: **Akerström, Bengt, Skiftesvägen 26,
S-183 38 Täby (SE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Endocard-Elektrodenanordnung

Die Erfindung betrifft eine Endocard-Elektrodenanordnung für die intrakardiale Stimulation des Herzens, mit einem langgestreckten elektrischen Leiter, der mit einer elektrischen Isolierung versehen ist, und mit einem mit dem distalen Ende des Leiters elektrisch leitend verbundenen, für die Zuführung der Stimulationsimpulse zum Herzen dienenden Elektrodenkopf und mit Mitteln zum Fixieren des Leiters bzw. des Elektrodenkopfes an der Herzwand.

Aus der US-Patentschrift 3 902 501 ist eine Endocard-Elektrode dieser Art bekannt. Als Fixiermittel dienen bei dieser Elektrode relativ steife Widerhaken aus Silicongummi unmittelbar hinter dem Elektrodenkopf, die nach Applikation ins Herzgewebe eingreifen und somit die Elektrode am Platz halten. Das Einführen einer solchen Elektrode in eine Vene, insbesondere mit einem kleinen Durchmesser, wie z.B. bei Kindern, bereitet jedoch wegen des relativ voluminösen Elektrodenkopfes nicht unerhebliche Schwierigkeiten. Bei vorhofgesteuerten Herzschrittmachern werden in der Regel sogar zwei Elektroden eingesetzt. Selbst bei Venen mit normal grossem Durchmesser sind zwei Elektrodenköpfe kaum noch plazierbar. Die Widerhaken erlauben auch kaum nachträgliche Korrekturen der Lage; ihr Anwuchs an die Herzwand ist erschwert, da sie dem Bindegewebe nur wenig Platz zum Umwachsen bieten.

Der Erfindung liegt die Aufgabe zugrunde, eine Endocard-Elektrodenanordnung der eingangs genannten Art zu schaffen, die gut am Herzen anwächst und die dennoch ein verhältnismässig kleines Volumen hat.

Diese Aufgabe ist erfindungsgemäss dadurch gelöst, dass zum Fixieren des Leiters bzw. des Elektrodenkopfes eine Schleife oder eine Mehrzahl solcher Schleifen aus weichem dünnem Material vorgesehen ist, die auf dem Elektrodenkopf oder auf der elektrischen Isolierung des langgestreckten elektrischen Leiters in der Nähe des Elektrodenkopfes befestigt ist.

Schleifen aus einem weichen dünnen Material legen sich beim Einführen der Elektrode in eine Vene auch mit sehr kleinem Durchmesser dicht an die Elektrode an. Der Einführung wird also kaum Widerstand entgegengebracht. Die Flexibilität des Schleifenmaterials erlaubt darüber hinaus Ortskorrekturen beliebiger Art, ohne dass das Trabekel beschädigt wird. In die Schleifen wächst ausserdem rasch und leicht Bindegewebe hinein, so dass die Elektrode an der Herzwand gut fixiert wird. Ein weiterer Vorteil der Schleifen liegt darin, dass die Schleifen beim Durchführen der Elektrode durch eine Vene mit Blut-Koagulat gefüllt werden. Das Blut-Koagulat dient als Kleber, so dass die Schleifen an der Herzwand festgeklebt werden, was zu einem besonders raschen Festwachsen der Elektrode führt.

Ein weiterer Vorteil ist darin zu sehen, dass die erfindungsgemässe Elektrode wegen ihres geringen Durchmessers bei einer Subclava-Punktion verwendet werden kann. Bei einer Subclava-Punktion wird ohne chirurgischen Schnitt ein Katheter kleinen Durchmessers durch die Subclava gestochen. Die Elektrode wird dann durch den Katheter in das Gefäss zum Herzen eingeführt.

Eine vorteilhafte Weiterbildung der Erfindung besteht darin, dass die Schleifen aus einem resorbierbaren Material bestehen. Dadurch ist erreicht, dass die eingepflanzte Elektrode zu einem späteren Zeitpunkt sehr leicht zu entfernen ist, da die Schleifen sich mit der Zeit auflösen.

Weitere Einzelheiten ergeben sich aus den Unteransprüchen.

Die Erfindung ist anhand mehrerer in den Figuren dargestellter Ausführungsbeispiele näher erläutert. Es zeigen:

Fig. 1 das distale Ende einer erfindungsgemässen Endocard-Elektrodenanordnung,

Fig. 2 und 3 Varianten der Endocard-Elektrodenanordnung gemäss Fig. 1,

Fig. 4 die Endocard-Elektrodenanordnung gemäss Fig. 3 in Vorderansicht,

Fig. 5 eine Variante der Endocard-Elektrodenanordnung nach Fig. 3.

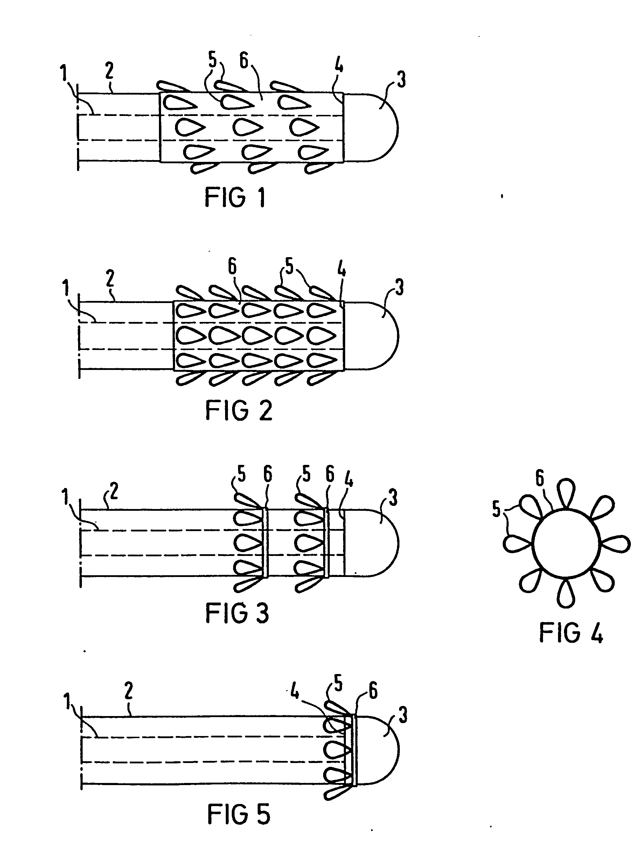

In Fig. 1 ist mit 1 der elektrische Leiter einer Elektrode bezeichnet. Er ist mit einer elektrischen Isolierung 2 versehen. Am distalen Ende befindet sich der Elektrodenkopf 3, der im wesentlichen zylindrisch ausgebildet und an seinem freien Ende abgerundet ist. Der Kopf kann auch andere Formen, z.B. reine ungerundete Zylinderform od. dgl., aufweisen. An seinem hinteren Ende 4 ist der Elektrodenkopf 3 mit dem elektrischen Leiter 1 verbunden. Nach Applizieren des Elektrodenkopfes 3 an der Herzwand können dem Herzen über Leiter 1 und Elektrodenkopf 3 Stimulationsimpulse zugeführt werden.

Damit die Elektrode an ihrem Platz im Herzen sicher gehalten wird, sind in der Nähe des Elektrodenkopfes Schleifen 5 vorgesehen, in die Herzgewebe einwachsen kann. Die Schleifen 5 sind in Fig. 1 auf einem Kragen 6, der auf die Isolierung 2 geschoben ist, befestigt. Hier sind die Schleifen 5 entlang einer wendelförmigen Linie angebracht.

Bei der Elektrode der Fig. 2 sind die Schleifen 5 auf dem Kragen 6 am Umfang verteilt entlang paralleler Linien, die in der Längsrichtung des langgestreckten Leiters 1 verlaufen befestigt.

Bei der Elektrode der Fig. 3 sind mehrere Kragen 6, die mit Schleifen 5 versehen sind, in Abstand voneinander auf die Isolierung 2 des Leiters 1 geschoben.

Die Elektrode der Fig. 4 zeigt den Kragen 6 mit Schleifen 5 in Vorderansicht. Diese Art der Ausbildung ermöglicht Ausstanzen als komplettes Stanzteil.

Die Elektrode der Fig. 5 zeigt eine Anordnung eines Kragens 6 mit Schleifen 5 auf dem Elektrodenkopf 3.

Die Schleifen 5 können aus einem weichen dünnen resistiven Material, wie z.B. Polyester oder Polypropylen, gefertigt werden. Ebensogut kann zur Fertigung auch ein resorbierbares Material, wie z.B. Katgut, eingesetzt werden, das sich bei einer eingepflanzten Elektrode mit der Zeit auflöst, so dass ein Elektrodenaustausch bei Bedarf ohne Komplikationen möglich ist. Die Schleifen können anstelle auf Kragen auch direkt auf der elektrischen Isolierung 2 angebracht werden. Sie überragen die Oberfläche der Elektroden mit etwa 1 bis 5 mm. Dadurch, dass die Schleifen 5 aus einem sehr weichen und dünnen Material bestehen, liegen sie unabhängig von Anzahl und Grösse beim Einführen der Elektrode in eine Vene dicht an der Elektrode an. Bei kleinstem Durchmesser der Elektrode ergibt sich kaum Widerstand beim Einschieben. In den Schleifen sammelt sich beim Einführen der Elektrode auch Blut-Koagulat, das als Kleber das Festwachsen der Elektrode an der Herzwand erleichtert.

## Patentansprüche

1. Endocard-Elektrodenanordnung für die intrakardiale Stimulation des Herzens, mit einem langgestreckten elektrischen Leiter, der mit einer elektrischen Isolierung versehen ist, und mit einem mit dem distalen Ende des Leiters elektrisch leitend verbundenen, für die Zuführung der Stimulationsimpulse zum Herzen dienenden Elektrodenkopf und mit Mitteln zum Fixieren des Leiters bzw. des Elektrodenkopfes an der Herzwand, dadurch gekennzeichnet, dass zum Fixieren des Leiters bzw. des Elektrodenkopfes eine Schleife (5) oder eine Mehrzahl solcher Schleifen (5) aus weichem dünnem Material vorgesehen ist, die auf dem Elektrodenkopf (3) oder auf der elektrischen Isolierung (2) des langgestreckten elektrischen Leiters (1) in der Nähe des Elektrodenkopfes (3) befestigt ist.

2. Endocard-Elektrodenanordnung nach Anspruch 1, dadurch gekennzeichnet, dass eine Mehrzahl von Schleifen (5) entlang einer wendelförmigen Linie um das Isoliermaterial (2) des langgestreckten elektrischen Leiters (1) angeordnet ist.

3. Endocard-Elektrodenanordnung nach Anspruch 1, dadurch gekennzeichnet, dass eine Mehrzahl von Schleifen (5) entlang paralleler Linien in Längsrichtung der Isolierung (2) des langgestreckten elektrischen Leiters (1) angeordnet ist.

4. Endocard-Elektrodenanordnung nach einem der Ansprüche 1 bis 3, dadurch gekennzeichnet, dass die Schleife oder eine Mehrzahl von Schleifen (5) direkt an der elektrischen Isolierung (2) des elektrischen Leiters (1) befestigt ist.

5. Endocard-Elektrodenanordnung nach einem der Ansprüche 1 bis 3, dadurch gekennzeichnet, dass die Schleife oder eine Mehrzahl von Schleifen (5) auf einem Kragen (6), der auf die Isolierung (2) des elektrischen Leiters (1) oder auf den Elektrodenkopf (3) geschoben ist, befestigt ist.

6. Endocard-Elektrodenanordnung nach einem der Ansprüche 1 bis 5, dadurch gekennzeichnet, dass die Schleifen (5) aus einem resistiven Material bestehen.

7. Endocard-Elektrodenanordnung nach einem der Ansprüche 1 bis 5, dadurch gekennzeichnet, dass die Schleifen (5) aus einem resorbierbaren Material bestehen.

8. Endocard-Elektrodenanordnung nach einem der Ansprüche 1 bis 6, dadurch gekennzeichnet, dass die Schleifen (5) im herausgezogenen Zustand an der Oberfläche der Elektrodenanordnung (1) zwischen 1 und 5 mm herausragen.

## Revendications

1. Dispositif d'électrode endocardiaque pour la stimulation intercardiaque du coeur, avec un conducteur électrique allongé et pourvu d'une isolation électrique, et avec une tête d'électrode reliée électriquement à l'extrémité distale du conducteur et servant à appliquer des impulsions de stimulation au coeur, et avec des moyens pour fixer le conducteur ou la tête d'électrode à la paroi cardiaque, caractérisé per le fait que pour fixer le conducteur ou la tête d'électrode à la paroi cardiaque, il est prévu une boucle (5) ou plusieurs de telles boucles (5) en un matériau mince et mou et qui sont fixées sur la tête d'électrode (3) ou sur l'isolant élecrique (2) du conducteur électrique allongé (1) dans le voisinage de la tête d'électrode (3).

2. Dispositif d'électrode endocardiaque selon la revendication 1, caractérisé per le fait que plusieurs boucles sont disposées le long d'une ligne hélicoïdale autour du matériau isolant (2) du conducteur électrique (1).

3. Dispositif d'électrode endocardiaque selon la revendication 1, caractérisé per le fait que plusieurs boucles sont disposées le long de lignes parallèles dans la direction longitudinale de l'isolant (2) du conducteur électrique allongé (1).

4. Dispositif d'électrode endocardiaque selon l'une des revendications 1 à 3, caractérisé per le fait que la boucle ou plusieurs boucles (5) sont fixées à l'isolant (2) du conducteur électrique.

5. Dispositif d'électrode endocardiaque selon l'une des revendications 1 à 3, caractérisé per le fait que la boucle ou plusieurs boucles (5) sont fixées sur une collerette (6) glissée sur l'isolant (2) du conducteur électrique (1) ou sur la tête d'électrode (3).

6. Dispositif d'électrode endocardiaque selon l'une des revendications 1 à 5, caractérisé per le fait que les boucles (5) sont constituées avec un matériau résistif.

7. Dispositif d'électrode endocardiaque selon l'une des revendications 1 à 5, caractérisé per le fait que les boucles sont constituées avec un matériau capable d'être résorbé.

8. Dispositif d'électrode endocardiaque selon l'une des revendications 1 à 6, caractérisé per le fait que les boucles (5) à l'état saillant débordent la surface du dispositif d'électrode entre 1 et 5 mm.

## Claims

1. An endocardiac electrode arrangement for the intracardial stimulation of the heart, comprising an elongate elctrical conductor, which is provided with an electrical insulation, an electrode head which is electrically connected to the distal end of the conductor and which serves to supply the stimulation pulses to the heart, and means for fixing the conductor or the electrode head, to the heart wall, characterised in that for fixing the conductor or the electrode head, a loop (5), or a plurality of such loops (5), is provided, said loop or loops being made of a soft, thin material and being fastened on the electrode head (3), or on the electrical insulation (2) of the elongate lectrical conductor (1) in the vicinity of the electrode head (3).

2. An endocardiac electrode arrangement according to claim 1, characterised in that a plurality of loops (5) are arranged along a helical line around the insulating material (2) of the elongate electrical conductor (1).

3. An endocardiac electrode arrangement according to claim 1, characterised in that a plurality of loops (5) are arranged along parallel lines in the longitudinal direction of the insulation (2) of the elongate electrical conductor (1).

4. An endocardiac electrode arrangement according to one of claims 1 to 3, characterised in that the loop, or plurality of loops (5), is or are directly secured to the electrical insulation (2) of the electrical conductor (1).

5. An endocardiac electrode arrangement according to one of claims 1 to 3, characterised in that the loop or a plurality of loops (5) is or are arranged on a collar (6) which is slipped onto the insulation (2) of the electrical conductor (1), or onto the electrode head (3).

6. An endocardiac electrode arrangement according to one of claims 1 to 5, characterised in that the loops (5) consist of a resistive material.

7. An endocardiac electrode arrangement according to one of claims 1 to 5, characterised in that the loops (5) consist of a resorbable material.

8. An endocardiac electrode arrangement according to one of claims 1 to 6, characterised in that the loops (5) project from the surface of the electrode arrangement (1) by between 1 and 5 mm in the pulled-out state.

FIG 1

FIG 2

FIG 3

FIG 4

FIG 5